# EUROPEAN PATENT APPLICATION

(11) **EP 4 442 264 A1**
(43) Date of publication of application: **09.10.2024**
(21) Application number: 22901419.6
(22) Date of filing: 01.12.2022
(51) Int. Cl.: A61K 35/741, A61K 35/74, A61P 1/12, C12N 1/20

(54) **THERAPEUTIC AGENT FOR DIARRHEA AND METHOD FOR TREATING BOVINE DIARRHEA**

(30) Priority: 02.12.2021 JP 2021196413
(71) Applicant: Tohoku University, Sendai-shi, Miyagi 980-8577 (JP); Chiba Prefectural Agricultural Mutual Aid Association, Chiba-shi, Chiba 260-0031 (JP)
(72) Inventor: NOCHI Tomonori, Sendai-shi, Miyagi 980-8577 (JP); ISLAM Jahidul, Sendai-shi, Miyagi 980-8577 (JP); TANAKA Hidekazu, Chiba-shi, Chiba 260-0031 (JP)
(74) Representative: Heyerhoff Geiger GmbH & Co. KG
(86) International application number: PCT/JP2022/044458
(87) International publication number: WO 2023/100989

(57) **Abstract**

Provided are a therapeutic agent for diarrhea and a method for treating bovine diarrhea, which have improved therapeutic effects on diarrhea. The object can be achieved by a therapeutic agent for diarrhea containing bacteria, in which the therapeutic agent for diarrhea contains at least one type of bacterium selected from the group consisting of 13 types of bacteria listed in Bacterial Classification Nos. 1 to 13 shown in Table 1 below, and the 13 types of bacteria listed in Bacterial Classification Nos. 1 to 13 each contain a sequence having 95% or more sequence identity to a DNA sequence encoding 16S rRNA represented by SEQ ID NOs: 1 to 13.

## Description

### Technical Field

The disclosure in the present application relates to a therapeutic agent for diarrhea and a method for treating bovine diarrhea.

### Background Art

There is known fecal microbiota transplantation (FMT) for treating diarrhea symptoms by transplanting feces derived from a healthy cow to a cow that develops intractable diarrhea. As a related technology, it is described in NPL 1 that an increase in Porphyromonadaceae Family is useful for FMT.

### Citation List

### Non Patent Literature

NPL 1: H. S. Kim et al., "Longitudinal evaluation of fecal microbiota transplantation for ameliorating calf diarrhea and improving growth performance", Nat. Commun. 12(1), 1-16 (2021)

### Summary of Invention

### Technical Problem

In order to successfully treat bovine diarrhea, the type of bacteria contained in the therapeutic agent (for example, feces) to be transplanted is important. However, for example, it is not known which type of bacteria is effective for a cow having diarrhea symptoms. Therefore, it is desired to discover bacteria excellent in the treatment of diarrhea symptoms.

The disclosure in the present application has been made to solve the above problems. The present inventors have conducted intensive studies and analyzed a combination of a donor providing feces and a recipient receiving feces transplantation in FMT by a success example and a failure example in more detail, thereby newly finding a type of bacterium useful for the treatment of diarrhea.

That is, an object of the present disclosure is to provide a therapeutic agent for diarrhea and a method for treating bovine diarrhea.

### Solution to Problem

(1) A therapeutic agent for diarrhea containing bacteria,
in which the therapeutic agent for diarrhea contains
at least one type of bacterium selected from the group consisting of 13 types of bacteria listed in Bacterial Classification Nos. 1 to 13 shown in Table 1 below, and
the 13 types of bacteria listed in Bacterial Classification Nos. 1 to 13 each contain a sequence having 95% or more sequence identity to a DNA sequence encoding 16S rRNA represented by SEQ ID NOs: 1 to 13.

**[Table 1]**

| SEQ ID NO | Bacteria | |
|---|---|---|
| | Bacterial Classification No. | Bacterial name |
| 1 | 1 | c_Bacteroidia |
| 2 | 2 | f Prevotellaceae |
| 3 | 3 | g_Prevotella |
| 4 | 4 | g_Succinispira |
| 5 | 5 | g_Selenomonas |
| 6 | 6 | g_Gemmiger |
| 7 | 7 | g_Odoribacter |
| 8 | 8 | g_Lactobacillus |
| 9 | 9 | o_Lactobacillales |
| 10 | 10 | f_Veillonellaceae |
| 11 | 11 | g_Acidaminococcus |
| 12 | 12 | g_Collinsella |
| 13 | 13 | g_Sporobacter |

(2) The therapeutic agent for diarrhea according to (1), in which the bacteria include at least one type selected from the group consisting of Bacterial Classification Nos. 1 to 5, 7, 10, and 13, and has an occupancy rate shown in Table 2 below.

**[Table 2]**

| SEQ ID NO | Bacteria | | Occupancy rate |
|---|---|---|---|
| | Bacterial Classification No. | Bacterial name | |
| 1 | 1 | c_Bacteroidia | 24.21% or more |
| 2 | 2 | f Prevotellaceae | 4.43% or more |
| 3 | 3 | g_Prevotella | 4.77% or more |
| 4 | 4 | g_Succinispira | 4.12% or more |
| 5 | 5 | g_Selenomonas | 0.09% or more |
| 7 | 7 | g_Odoribacter | Greater than 0% |
| 10 | 10 | f_Veillonellaceae | 4.86% or more |
| 13 | 13 | g_Sporobacter | 0.16% or more |

(3) The therapeutic agent for diarrhea according to (1) or (2), containing at least g_Sporobacter of Bacterial Classification No. 13.
(4) The therapeutic agent for diarrhea according to (3), in which an occupancy rate of g_Sporobacter of Bacterial Classification No. 13 is 0.21% or more.
(5) The therapeutic agent for diarrhea according to (1) or (2), containing at least g_Selenomonas of Bacterial Classification No. 5.
(6) The therapeutic agent for diarrhea according to (5), in which an occupancy rate of g_Selenomonas of Bacterial Classification No. 5 is 1.78% or more.
(7) The therapeutic agent for diarrhea according to any one of (1) to (6), which is used for treatment of bovine diarrhea.
(8) The therapeutic agent for diarrhea according to any one of (1) to (7), further containing at least one type of compound selected from the group consisting of 7-methylguanine, N6-acetyllysine, Taurine, GABA, Ala-Ala, Creatine, N1-acetylspermidine, and Glu.
(9) The therapeutic agent for diarrhea according to any one of (1) to (8), in which the therapeutic agent for diarrhea is to increase at least one type of bacterium selected from the group consisting of g_Selenomonas of Bacterial Classification No. 5, f_Veillonellaceae of Bacterial Classification No. 10, g_Acidaminococcus of Bacterial Classification No. 11, and g_Collinsella of Bacterial Classification No. 12, which are contained in feces of a recipient after administration.
(10) The therapeutic agent for diarrhea according to any one of (1) to (9), in which the therapeutic agent for diarrhea is in a freeze-dried form.
(11) A method for treating bovine diarrhea, the method including: a step of administering the therapeutic agent for diarrhea according to any one of (1) to (10) into a bovine intestinal tract.

### Advantageous Effects of Invention

It is possible to improve a therapeutic effect on diarrhea by a therapeutic agent for diarrhea and a method for treating bovine diarrhea disclosed in the present application.

### Brief Description of Drawings

[FIG. 1] FIG. 1 is a graph showing results of FMT in Example 1.
[FIG. 2] FIG. 2 is a graph showing analysis results of bacteria included in a donor before FMT implementation (day 0) in Example 2.
[FIG. 3] FIG. 3 is a graph showing analysis results of bacteria included in a recipient before FMT implementation (day 0) in Example 2.
[FIG. 4] FIG. 4 is a graph showing analysis results of bacteria included in the recipient after FMT implementation (day 7) in Example 2.
[FIG. 5A] FIG. 5A is a graph showing a relative abundance of each bacterium in all bacteria in feces collected from a donor and a recipient (day 0, after day 1, and after day 7) of a success group, and a relative abundance of each bacterium in all bacteria in feces collected from a donor and a recipient (day 0, after day 1, and after day 7) of a failure group, in Example 3.
[FIG. 5B] FIG. 5B is a graph showing a relative abundance of each bacterium in all bacteria in feces collected from a donor and a recipient (day 0, after day 1, and after day 7) of a success group, and a relative abundance of each bacterium in all bacteria in feces collected from a donor and a recipient (day 0, after day 1, and after day 7) of a failure group, in Example 3.
[FIG. 6] FIG. 6 is a graph showing results of LEfSe analysis in Example 4.
[FIG. 7] FIG. 7 is a graph showing a relative abundance of g_Sporobacter in all bacteria in feces collected from a healthy cow, a cow with diarrhea, a donor and a recipient of a success group (day 0, after day 1, and after day 7), and a donor and a recipient of a failure group (day 0, after day 1, and after day 7), in Example 4.
[FIG. 8] FIG. 8 is a graph showing a VIP score of metabolite analyzed by CE-TOFMS metabolomics analysis in Example 5.
[FIG. 9] FIG. 9 is a graph showing a summary of results of metagenomic analysis in Example 6.
[FIG. 10] FIG. 10 is a graph showing detailed results of the metagenomic analysis in Example 6.
[FIG. 11] FIG. 11 is a graph showing detailed results of the metabolomics analysis in Example 6.

### Description of Embodiments

Hereinafter, the therapeutic agent for diarrhea and the method for treating bovine diarrhea, which are disclosed in the present application, will be described in detail. In the present description, a numerical value, a numerical value range, and a qualitative expression (for example, expression of "the same" or "identical") indicate a numerical value, a numerical value range, and a property including an error generally allowed in the technical field.

### (Embodiment of therapeutic agent for diarrhea)

The therapeutic agent for diarrhea disclosed in the present application contains at least one type of bacterium selected from the group consisting of 13 types of bacteria listed in Bacterial Classification Nos. 1 to 13 in Table 3 below (hereinafter, Bacterial Classification No. is simply described as "No."). The bacteria listed in No. 1 include a sequence having 95% or more sequence identity to a DNA sequence encoding 16S rRNA represented by SEQ ID NO: 1. The sequence of the bacteria listed in No. 1, which is the same as SEQ ID NO: 1, may be 96% or more, 97% or more, 98% or more, 99% or more, or 100%. The bacteria listed in No. 2 include a sequence having 95% or more sequence identity to a DNA sequence encoding 16S rRNA represented by SEQ ID NO: 2. The sequence of the bacteria listed in No. 2, which is the same as SEQ ID NO: 2, may be 96% or more, 97% or more, 98% or more, 99% or more, or 100%. Similarly, the bacteria listed in Nos. 3 to 13 each include a sequence having 95% or more sequence identity to a DNA sequence encoding 16S rRNA represented by SEQ ID NOs: 3 to 13. The sequence, which is the same as SEQ ID NO: 3 to 13 included in respective bacteria listed in Nos. 3 to 13, may be 96% or more, 97% or more, 98% or more, 99% or more, or 100%. At least one type of the bacteria listed in Nos. 1 to 13 may be contained in the therapeutic agent for diarrhea, and a plurality of bacteria, for example, two or more, three or more, four or more, and the like, may be combined. In addition, all of the 13 types of bacteria listed in Nos. 1 to 13 may be contained. In Table 3, "p" at the beginning of the bacterial name means phylum, "c" means class, "o" means order, "f" means family, and "g" means genus.

**[Table 3]**

| SEQ ID NO | Bacteria | |
|---|---|---|
| | Bacterial Classification No. | Bacterial name |
| 1 | 1 | c Bacteroidia |
| 2 | 2 | f Prevotellaceae |
| 3 | 3 | g_Prevote la |
| 4 | 4 | g_Succinispira |
| 5 | 5 | g_Selenomonas |
| 6 | 6 | g_Gemmiger |
| 7 | 7 | q Odoribacter |
| 8 | 8 | g_Lactobacillus |
| 9 | 9 | o Lactobacillales |
| 10 | 10 | f Veillonellaceae |
| 11 | 11 | g_Acidamnococcus |
| 12 | 12 | g_Colinsela |
| 13 | 13 | g_Sporobacter |

**[Table 4]**

| SEQ ID NO | DNA sequence encoding 16S rRNA |
|---|---|
| 1 | ATCCTGTTTGATCCCCACGCTTTCGTGCATCAGCGTCAATTGCGGATTCGTGAGATGCCTTCGCAATCGGTGTTCTGTGTGATATCTATGCATTTCACCGCTACACCACACATTCCTCCCACGGCATCCGCATT CTAGCCTACCAGTATCAACGGCACGGTAGAGGTTGAGCCCCTAAATTTCACCGCTGACTTAATAGACCGCCTACGCACCCTTTAAACCCAATAAATCCGGATAACGCTTGCATCCCCCGTATTACCGCGGCTGC TGGCACGGAGTTAGCCGATGCTTATTCGTAAGGTACTCTCATCAGCCTACACGTAGACCTTATTGCTCCCTTACAAAAGAAGTTTAAGACCCATAGGGCCGTCACCTTCACGCGGCATGGCTGGTTCAGTCGTC AGACCATTGACCAATATTCCTCA |
| 2 | AATCCTGTTCGATACCCGCGCTTTCGAGCTTCAGCGTCAGTTGCGCCCCAGTGAGCTGCCTTCGCAATCGGAGTTCTTCGTGATATCTAAGCATTTCACCGCTACACCACGAATTCCGCCCACTTTATGCGTA CTCAAGGAAACCAGTTCGCGCTGCAGTGCAGACGTTGAGCGTCTACATTTCACAACACGCTTAATCTCCGGCCTACGCTCCCTTTAAACCCAATAAATCCGGATAACGCCCGGACCTTCCGTATTACCGCGGC TGCTGGCACGGAATTAGCCGGTCCTTATTCATAGGGTACATGCAAAAAACCACACGTGGCTCACTTTATTCCCCTATAAAAGCAGTTTACAACCCATAGGGCCGTCTTCCTGCACGCTACTTGGCTGGTTCAGG CTCTCGCCCATTGACCAATATTCCTCA |
| 3 | ATCCTGTTCGATACCCGCACTTTCGAGCTTCAGCGTCAGTTGCGCTCCAGTGAGCTGCCTTCGCAATCGGAGTTCTTCGTGATATCTAAGCATTTCACCGCTACACCACGAATTCCGCCCACTTTGTGCGTACT CAAGGAAACCAGTTCGCGCTGCAGTGCAGACGTTGAGCGTCTACATTTCACAACACGCTTAATCTCCGGCCTACGCTCCCTTTAAACCCAATAAATCCGGATAACGCCCGGACCTTCCGTATTACCGCGGCTG CTGGCACGGAATTAGCCGGTCCTTATTCATAAGGTACATGCAAAAAGTCTCACGAGACTCACTTTATTCCCTTATAAAAGCAGTTTACAACCCATAGGGCCGTCTTCCTGCACGCTACTTGGCTGGTTCAGGCTC TCGCCCATTGACCAATATTCCTCA |
| 4 | AATCCCGTTCGCTACCCTGGCTTTCGCATCTCAGCGTCAGACACAGTCCAGAAAGGCGCCTTCGCCACCGGTGTTCTTCCCAATATCTACGCATTTCACCGCTACACTGGGAGTTCCCCTTTCCTCTCCTGCA CTCAAGTCCCCCAGTTTCGATCGGCATACGGCGTTAAGCACCGCATTTTCACGACCGACTTAAGGAACCGCCTACATGCGCTTTACGCCCAATAATTCCGGACAACGCTTGCCACCTACGTATTACCGCGGCT GCTGGCACGTAGTTAGCCGTGGCTTCCTCGTTCACTACCGTCACTGGAAACCCATTTCCTGGTCCCCCCGTTCGTCGTGAACAACAGAGTTTTACGTTCCGAAGAACTTCATCACTCACGCGGCGTTGCTCCG TCAGGCTTTCGCCCATTGCGGAAGATTCCCCA |
| 5 | ATCCTGTTCGCTCCCCACGCTTTCGAGCCTCAGCGTCAGTTACAGTCCAGAAAGCCGCCTTCGCCACTGGTGTTCCTCCTAATATCTACGCATTTCACCGCTACACTAGGAATTCCGCTTTCCTCTCCTGCAC TCAAGAAAGATAGTTTCCATCCCCTCACGGGGTTGAGCCCCGCACTTTTAAGATAGACTTACCTTCCCGCCTGCGCTCCCTTTACGCCCAATAATTCCGGACAACGCTCGCCACCTACGTATTACCGCGGCTG CTGGCACGTAGTTAGCCGTGGCTTCCTCGTCAGGTACCGTCATTCAGAACCCATATTCTGAGTCCTGACATTCGTCCCCGACAACAGAGCTTTACGAGTCCAAACCCTTCTTCACTCACGCGGCGCCTTGCTCCG TCAGGCTTGCGCCCATTGCGGAAGATTCCCCAC |
| 6 | ATCCTGTTTGCTACCCATGCTTTCGAGCCTCAGCGTCAGTTGGTGCCCAGTAGGTCGCCTTCGCCACTGGTGTTCCTCCCGATATCTACGCATTCCACCGCTACACCGGGAATTCCACCTACCTCTGCACTA CTCAAGGCCAGCAGTTTTGAAAGCAATTTATGGGTTGAGCCCATAGTTTTCACTTCCAACTTGCCGGTCCGCCTGCGCTCCCTTTACACCCAGTAATTCCGGACAACGCTTGCACCCTACGTTTTACCGCGGCT GCTGGCACGTAGTTAGCCGGTGCTTTCTTCTGTTAGGTACCGTCATTATCGTCCCTAACGACAGGAGTTTACAATCCGAAAACCTTCTTCCTCCACGCGGCGTCGCTGCATCAGGGTTTCCCCCATTGTGCAATAT CCCCCAC |
| 7 | ATCCTGTTCGCTACCCACGCTCTCGTGCTTCAGCGTCAGTCACAGTCTGGTAAGCTGCCTTCGCTATCGGAGTTCTAAGTTATATCTAAGCATTTCACCGCTACTTAACTTATTCCGCCTACCTCGTCTGTACT CAAGCTGACCAGTATTAATGGCTGGCCAAGGTTTAGCCATGACATTTTACCACTAACTTAATCAACCGCCTACGCACCCTTTAAACCCAATAAATCCGGATAACGCTCGCATCCTCCGTATTACCGCGGCTGCTG GCACGGAGTTAGCCGATGCTTATTCATTCGGTACAGGCATCGTTCCACGCGTGAAACTTATTCTTCCCGAATAAAAGAGGTTTACAACCCATAGGGCAGTCTTCCCTCACGCGACTTGGCTGGTTCAGCCTTCC GGCCATTGACCAATATTCCTCAC |
| 8 | ATCCTGTTTGCTACCCACGCTTTCGAACCTCAGCGTCAGTTACAGACCAGAGAGCCGCTTTCGCCACTGGTGTTCTTCCATATATCTACGCATTTCACCGCTACACATGGAGTTCCACTCTCCTCTTCTGCACT CAAGTCTTCCAGTTTCCAATGCACTACTCCGGTTAAGCCGAAGGCTTTCACATCAGACTTAAAAGACCGCCTGCGTTCCCTTTACGCCCAATAAATCCGGACAACGCTTGCCACCTACGTATTACCGCGGCTGC TGGCACGTAGTTAGCCGTGACTTGCTGGTTAGATACCGTCATCGAATGAACAGTTACTCTCACTCGTGTTCTTCTCTAACAACAGAGTTTTACGATCCGAAGACCTTCTTCACTCACGCGGCGTTGCTCCATCAG ACTTGCGTCCATTGTGGAAGATTCCCTA |
| 9 | ATCCTGTTCGCTCCCCACGCTTTCGAGCCTCAGCGTCAGTTACAGACCAGAGAGCCGCCTTCGCCACTGGTGTTCCTCCATATATCTACGCATTTCACCGCTACACATGGAATTCCACTCTCCTCTTCTGCAC TCAAGTCTCCCAGTTTCCAATGACCCTCCCCGGTTGAGCCGGGGGCTTTCACATCAGACTTAAGAAACCGCCTGCGCTCGCTTTACGCCCAATAAATCCGGACAACGCTTGCCACCTACGTATTACCGCGGCT GCTGGCACGTAGTTAGCCGTGGCTTTCTGGTTAGATACCGTCAAGGGATGAACATTCTCTTCTCATCCTTGTTCTTCTCTAACAACAGAGTTTTACGATCCGAAAACCTTCTTCACTCACGCGGCGTTGCTCGGT CAGACTTTCGTCCATTGCCGAAGATTCCCTACTG |
| 10 | AATCCCGTTCGCTACCCTGGCTTTCGCATCTCAGCGTCAGACACAGTCCAGAAAGGCGCCTTCGCCACCGGTGTTCTTCCCAATATCTACGCATTTCACCGCTACACTGGGAGTTCCCCTTTCCTCTCCTGCA CTCAAGTGCCACAGTTTCCATCGCCATACGGGGTTAGGCCCCGCAGTTTCACGACAGACTTAAGGCACCGCCTACATGCGCTTTACGCCCAATAATTCCGGACAACGCTTGCCACCTACGTATTACCGCGGCT GCTGGCACGTAGTTAGCCGTGGCTTCCTCGTACACTACCGTCATTGCTTCCCGTTGTTGATAGAAAGCACGTTCGTCATGTACAACAGGGCTTTACGTTCCGAAGAACTTCCTCACCCACGCGGCGTTGCTCC GTCAGGCTTTCGCCCATTGCGGAAGATTCCCCA |
| 11 | AATCCCGTTTGCTACCCTGGCTTTCGCATCTCAGCGTCAGACACAGTCCAGAAAGCCGCCTTCGCCACTGGTGTTCCTCCCAATATCTACGCATTTCACCGCTACACTGGGAATTCCCCTTTCCTCTCCTGCA CTCAAGACTTCCAGTATCCAACGCCATACGGGGTTAAGCCCCGCATTTTCACGTCGGACTTAAAAGCCCGCCTACATGCTCTTTACGCCCAATAATTCCGGACAACGCTTGCCACCTACGTATTACCGCGGCT GCTGGCACGTAGTTAGCCGTGGCTTCCTCGTCAGGTACCGTCAACACCAAAATGTATTATAGATCGGTGCTTTCGTCCCTGACAACAGAGTTTTACAACCCGAAGGCCTTCATCACTCACGCGGCGTTGCTCC GTCAGACTTTCGTCCATTGCGGAAGATTCCCCA |
| 12 | AATCCTGTTCGCTCCCCCAGCTTTCGCGCCTCAGCGTCGGTCTCGGCCCAGAGGGCCGCCTTCGCCACCGGTGTTCCACCCGATATCTGCGCATTCCACCGCTACACCGGGTGTTCCACCCTCCCCTACC GGACCCAAGCCCGGAGGTTCCGGGGGCTTCGGGGGGTTGAGCCCCCCGCTTCGACCCCCGGCCTGCCGGGCCGCCTACGCGCGCTTTACGCCCAATGAATCCGGATAACGCTCGCCCCCTACGTATTA CCGCGGCTGCTGGCACGTAGTTAGCCGGGGCTTCTTCTGCAGGTACAGTCTTGACTCTTCCCTGCTGAAAGCGGTTTACGACCCGAAGGCCTCCATCCCGCACGCGGCGTCGCTGCGTCAGGGTTGCCCC CATTGCGCAAGATTCCCCA |
| 13 | AATCCTGTTTGCTCCCCACGCTTTCGCGCCTCACCGTCAGTTGTCGTCCAGTCATCCGCCTTCGCCACTGGTGTTCTTCCTTATATCTACGCATTTCACCGCTACACAAGGAATTCCGATGACCTCTCCGATA CTCAAGAGAAACAGTTTCAAATGCAGTTTGGAGGTTGAGCCTCCAGATTTCACATCTGACTTGTCTCCCCGGCTACACGCCCTTTACACCCAGTAAATCCGGACAACGCTTGCCACCTACGTATTACCGCGGCT GCTGGCACGTAGTTAGCCGTGGCTTGTTTTCAGGGTACCGTCTTCTGCTCTTCCCCTGTCAAAGAAGTTTACAACCCGAAAGCCTTCTTCCTTCACGCGGCGTTGCTGGGTCAGACTTGCGTCCATTGCCCAA TATTCCCCA |

The therapeutic agent for diarrhea is not particularly limited as long as the therapeutic agent for diarrhea contains at least one type selected from the group consisting of the bacteria listed in Nos. 1 to 13. For example, the therapeutic agent for diarrhea is obtained by first sorting out a donor containing a large amount of the bacteria listed in Nos. 1 to 13 and collecting feces from the sorted donor. In addition, the therapeutic agent for diarrhea may be prepared by adding the bacteria listed in Nos. 1 to 13, which have been separately cultured, to feces collected from the sorted donor or the donor in a healthy state (that is, having no symptom of diarrhea). In addition, the therapeutic agent for diarrhea may be used as a liquid by diluting the collected feces with a buffer solution, physiological saline, or the like, or may be used as a powder by freeze-drying.

The therapeutic agent for diarrhea may be prepared based on feces obtained from a donor, or may be prepared without using feces of the donor. For example, at least one type of bacterium selected from the bacteria listed in Nos. 1 to 13 may be cultured to be proliferated, and the culture solution may be used as a therapeutic agent for diarrhea. In addition, in a case where the bacteria proliferated by culturing are used, for example, the bacteria may be used in the form of a powder by freeze-drying.

In addition, regardless of whether the feces collected from the donor is used, the therapeutic agent for diarrhea disclosed in the present application may be used as a desired dosage form within a range that does not affect the effects disclosed in the present application, and may contain a carrier, an excipient, a diluent, and the like. Examples of the dosage form of the therapeutic agent for diarrhea include a liquid preparation, a capsule preparation, a granule preparation, a pill preparation, a powder preparation, a suspension/emulsion, and a suppository.

Examples of the carrier, the excipient, and the diluent include lactose, dextrose, sucrose, sorbitol, mannitol, starch, gum Arabic, calcium phosphate, alginate, Tragacanth, gelatin, calcium silicate, microcrystalline cellulose, polyvinylpyrrolidone, cellulose, water, phosphate buffered saline (PBS), syrup, methylcellulose, methyl- and propyl p-oxybenzoate, talc, and magnesium stearate, mineral oil. In addition, a vitamin, a lubricant, a humectant, an emulsifier and a suspending agent, a preservative, a sweetener, or a fragrance may be added. In addition, the therapeutic agent for diarrhea may be used in combination with a known drug.

As shown in Examples to be described later, the bacteria listed in Nos. 1 to 13 were contained in a larger amount in the donor of the success group than in the donor of the failure group of FMT. Therefore, it is desirable that the bacteria listed in Nos. 1 to 13 occupy a large proportion of a bacterial community in the therapeutic agent for diarrhea. Although not limited, the following occupancy rates are exemplified as the occupancy rates of the bacteria listed in Nos. 1 to 5, 7, 10, and 13. In the present description, the "occupancy rate" means a ratio of the number of bacteria of each of the types listed in Nos. 1 to 5, 7, 10, and 13 to the total number of bacteria contained in the therapeutic agent for diarrhea. In addition, the occupancy rate is a comparison at the same level (for example, class, order, family, genus) in bacterial classification. For example, the occupancy rate of No. 1 (c_Bacteroidia) is a ratio of No. 1 (c_Bacteroidia) in a case where all bacteria are classified at the class level. Similarly, the occupancy rate of No. 2 (f_Prevotellaceaec) is a ratio of No. 2 (f_Prevotellaceaec) in a case where all bacteria are classified at the family level, and the occupancy rate of No. 3 (g_Prevotella) is a ratio of No. 3 (g_Prevotella) in a case where all bacteria are classified at the genus level.
· No. 1 (c_Bacteroidia): 24.21% or more, preferably 31.34% or more, and more preferably 38.47% or more.
· No. 2 (f_Prevotellaceae): 4.43% or more, preferably 12.64% or more, and more preferably 20.85% or more.
· No. 3 (g_Prevotella): 4.77% or more, preferably 15.89% or more, and more preferably 27.00% or more.
· No. 4 (g_Succinispira): 4.12% or more, preferably 5.75% or more, and more preferably 7.40% or more.
· No. 5 (g_Selenomonas): 0.09% or more, preferably 1.78% or more, and more preferably 3.47% or more.
· No. 7 (g_Odoribacter): 0% or more (greater than 0), preferably 0.46% or more, and more preferably 0.92% or more.
No. 10 (f_Veillonellaceae): 4.86% or more, preferably 10.32% or more, and more preferably 15.78% or more.
· No. 13 (g_Sporobacter) : 0.16% or more, preferably 0.21% or more, and more preferably 0.28% or more.

In addition, the description of the occupancy rate of the bacteria of Nos. 6, 8, 9, 11, and 12 is omitted for convenience of the analysis, but the same preferable range as the bacteria listed in Nos. 1 to 5, 7, 10, and 13 may be determined.

As described in Examples to be described later, among the bacteria listed in Nos. 1 to 13, the genus Selenomonas of No. 5 and the genus Sporobacter of No. 13 are considered to be particularly preferable bacteria. Therefore, the therapeutic agent for diarrhea may be prepared so that the genus Selenomonas of No. 5 and/or the genus Sporobacter of No. 13 must be included. In addition, the occupancy rate of the genus Selenomonas of No. 5 may be 1.78% or more, and the occupancy rate of the genus Sporobacter of No. 13 may be 0.21% or more.

In addition, as described in Examples to be described later, in a case where the success group and the failure group were compared, the metabolites (compounds) contained in a large amount in the success group were specified. Specific examples thereof include 7-methylguanine, N6-acetyllysine, Taurine, γ-aminobutyric acid (GABA), Ala-Ala, Creatine, N1-acetylspermidine, and glutamic acid (Glu). The therapeutic agent for diarrhea disclosed in the present application may contain at least one type of compound selected from the group consisting of 7-methylguanine, N6-acetyllysine, Taurine, γ-aminobutyric acid (GABA), Ala-Ala, Creatine, N1-acetylspermidine, and glutamic acid (Glu), depending on the necessity.

The therapeutic agent for diarrhea is useful for any animals including livestock and poultry (for example, cattle, sheep, goats, pigs, and chickens), domestic pets (for example, dogs, cats, and rodents), and racehorses and edible horses.

As described above, the therapeutic agent for diarrhea disclosed in the present application preferably contains bacteria listed in Nos. 1 to 13 in a large amount. On the other hand, as shown in Examples to be described later, the bacteria listed in Nos. 14 to 64 shown in Tables 5A and 5B below are not preferable as the bacteria to be included in the therapeutic agent for diarrhea. Therefore, it is preferable that the bacteria listed in Nos. 14 to 64 have a lower occupancy rate in the therapeutic agent for diarrhea. In a case where the therapeutic agent for diarrhea is based on feces of a donor, the feces may be collected by selecting a donor having a low occupancy rate of the bacteria listed in Nos. 14 to 64. In a case where the therapeutic agent for diarrhea is prepared using cultured bacteria, the bacteria listed in Nos. 14 to 64 may not be used. In a case where the bacteria listed in Nos. 14 to 64 are used from a viewpoint of adjusting the bacterial community (intestinal flora), the occupancy rate in the therapeutic agent for diarrhea may be adjusted to be low. In the bacteria listed in Nos. 14 to 64, the occupancy rate of at least one type may be lowered, or the occupancy rates may be lowered by combining two or more. In addition, the bacteria listed in Nos. 14 to 64 are bacteria specified by the DNA sequence encoding 16S rRNA represented by SEQ ID NOS: 14 to 64, respectively, similar to the bacteria listed in Nos. 1 to 13. In Tables 5A and 5B below, there is a case where the same bacterial name may be assigned with different Bacterial Classification Nos. and different SEQ ID NOs (for example, No. 16 and No. 25 are assigned to "p_Tenericutes") . However, in Tables 5A and 5B below, even in a case where Bacterial Classification Nos. and the SEQ ID NOs are different from each other, when the bacterial names are the same, the bacteria are the same type of bacteria.

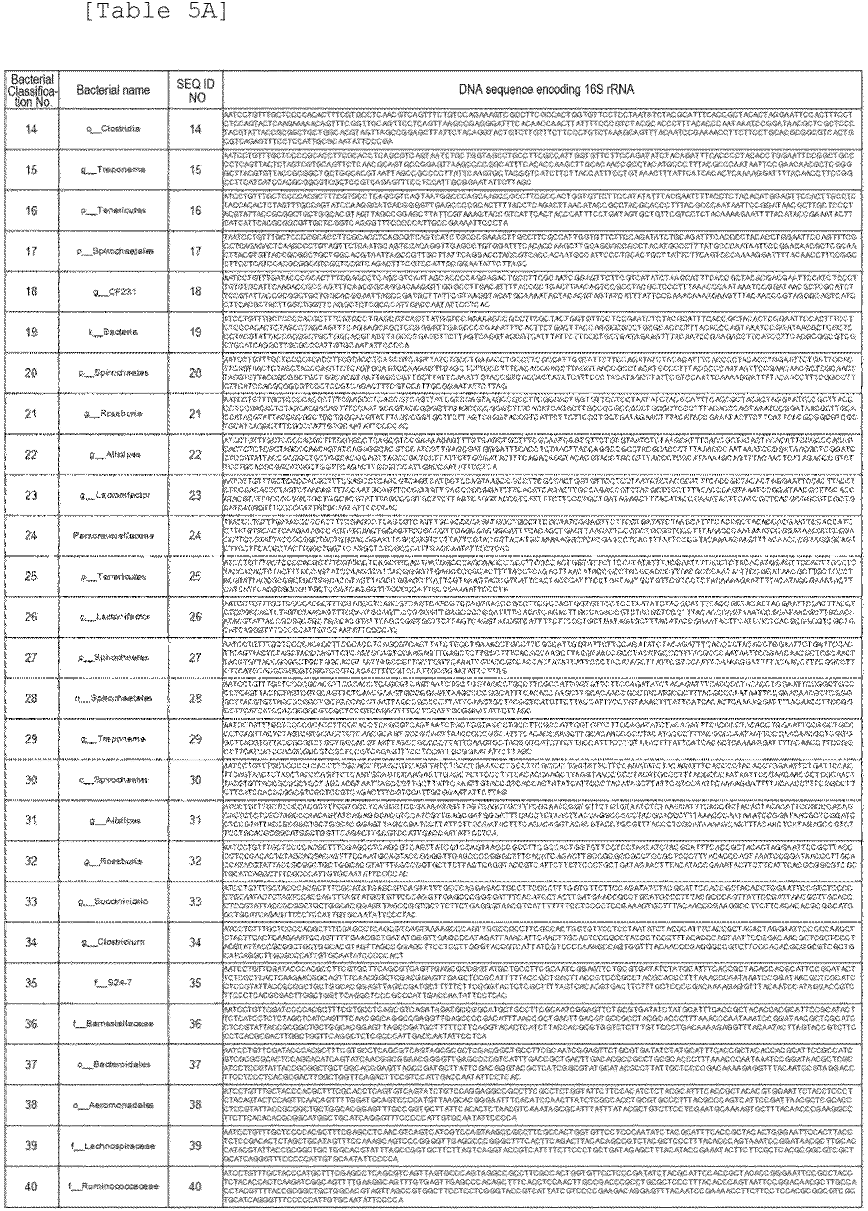

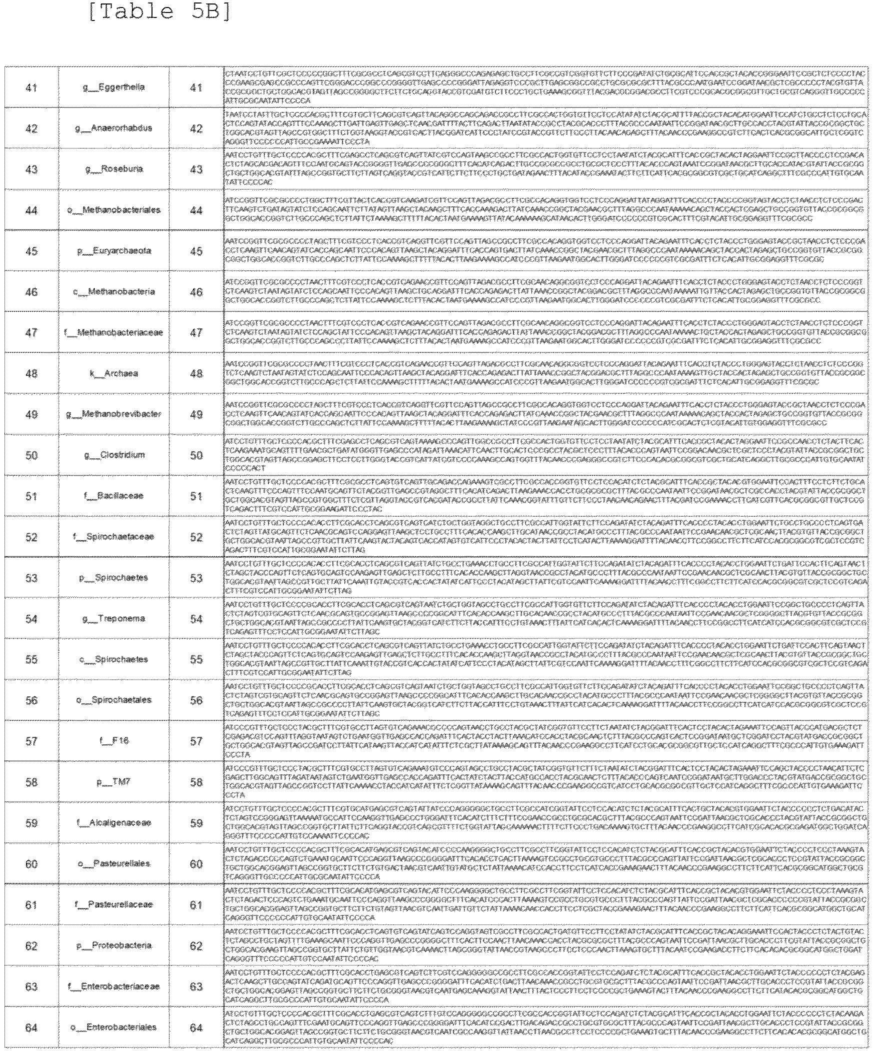

### (Embodiment of method for treating bovine diarrhea)

An embodiment of a method for treating bovine diarrhea disclosed in the present application includes a step of administering the above-described therapeutic agent for diarrhea into a bovine intestinal tract (for example, colon). The therapeutic agent for diarrhea may be administered to a cow showing diarrhea symptoms, for example, from the anus. In addition, the therapeutic agent for diarrhea may be administered orally.

Embodiments disclosed in the present application will be described below in detail with reference to Examples, but the examples are merely for describing embodiments. This does not represent limitation or restriction of the scope of the invention disclosed in the present application. Examples

### <Example 1>

FMT was carried out by the procedure shown below.

### [Animal]

In Examples, a total of 198 calves, including healthy calves and calves with diarrhea, raised in 20 farms in Chiba Prefecture, were used. Healthy calves (n = 20) and calves suffering from intractable diarrhea (n = 20) were randomly selected as donors and recipients of FMT. The intractable diarrhea is a diarrhea that cannot be cured even by carrying out various treatments. The severity of diarrhea in the recipient was scored as follows depending on the state of feces (diarrheal score).
1 = normal (shape of feces is maintained).
2 = soft (there is flow crossing surface).
3 = muddy (liquid state).
4 = severe diarrhea (very aqueous).

The fecal samples collected from FMT donors and recipients were subjected to pathogenicity evaluation for 10 major enteric pathogens that often cause causes of diarrhea (that is, bovine leukemia virus, bovine viral diarrhea, rotavirus, C. perfringens, C. parvum, coccidia, Salmonella, coronavirus, pathogenic Escherichia coli, and nematodes).

In order to estimate the diarrhea state and the large intestinal motility, an amount of water contained in the feces was examined by comparing weights before and after freeze-drying using the following calculation. · Water content (%) = 100 × (wet weight - dry weight)/wet weight

### [FMT procedure]

Fresh feces (up to 100 g) were collected from a healthy donor and suspended in 200 to 250 ml of sterile physiological saline (therapeutic agent for diarrhea). Large particles were filtered from a fecal suspension using medical gauze and immediately administered into the rectum of the recipient suffering from intractable diarrhea. The fecal samples were collected from the donors on the day of FMT, and were collected from the recipients before FMT implementation (day 0), after 1 day of FMT, and after 7 days of FMT. The collected fecal samples were stored in Stool Nucleic Acid Collection and Preservation Tubes (Norgen) at room temperature for metagenomic analysis. The present experiment was carried out based on the guidelines (Laboratory Animal Welfare and Animal Experiment Control) issued by the ethics committee of the Chiba Prefecture Agricultural Mutual Aid Association (approval numbers CNS190901 and CNS190902).

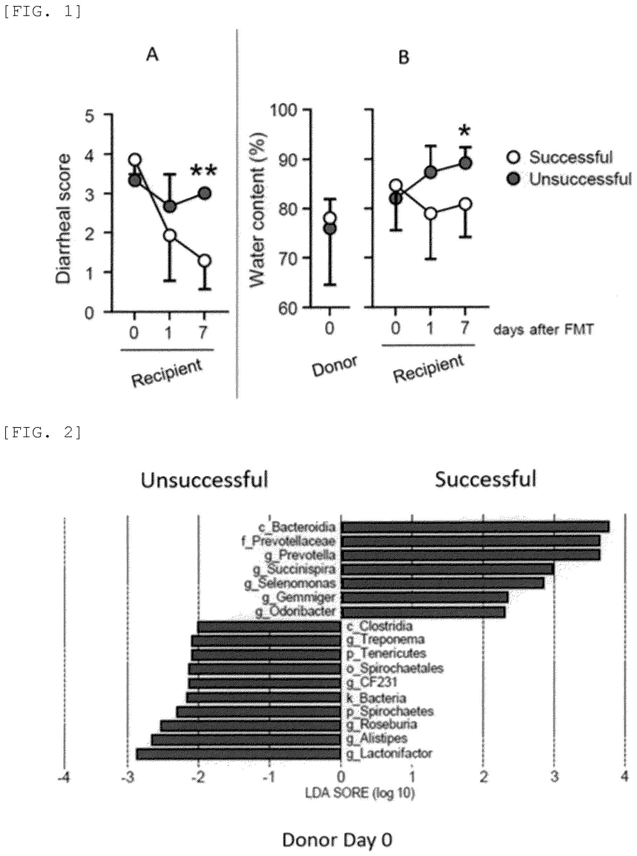

FIG. 1 shows the results of FMT. Effects of FMT were comprehensively determined based on the diarrheal score, the appearance of the body, and the state of the intestinal pathogenic bacteria contained in the feces collected from the recipient on day 0 and after 7 days. Among 20 calves subjected to FMT, 14 calves showed a therapeutic effect (success group: Successful), but 6 calves did not show a therapeutic effect (failure group: Unsuccessful). FIG. 1A shows a diarrheal score, in which a great therapeutic effect was observed in the success group after 1 day of FMT, and the diarrheal score was close to a normal value after 7 days. On the other hand, in the failure group, the therapeutic effect was observed slightly after 1 day of FMT, but the symptoms were deteriorated to almost the same as those on day 0 and after 7 days. In addition, as shown in FIG. 1B, the amount of water in the feces of the donor was almost the same, but the amount of water in the feces of the failure group of FMT was increased.

### <Example 2>

As shown in FIG. 1, the effect was not observed in about 30% of the recipients who were subjected to FMT, although all the donors were in a healthy state. Therefore, the types of bacteria that lead to success of FMT and the types of bacteria that lead to failure of FMT were analyzed with regard to the combination of the donor and the recipient in the success group and the combination of the donor and the recipient in the failure group by linear discriminant analysis effect size (LEfSe). In addition, the types of metabolites that lead to the success and lead to the failure of FMT were analyzed. The experimental procedure will be described below.

### [Amplification of 16S rRNA gene by PCR]

Genomic DNA was extracted from a fecal sample collected using a Stool DNA Isolation Kit according to the manufacturer's protocol. PCR was carried out by a known method (T. Nochi et al., "Elucidation of the Effects of a Current X-SCID Therapy on Intestinal Lymphoid Organogenesis Using an In Vivo Animal Model", Cell. Mol. Gastroenterol. Hepatol., 10 (1), 83-100 (2020)).

### [Metagenomic analysis and functional prediction analysis]

The reverse-multiplexed raw sequence was acquired from BaseSpace Sequence Hub (Illumina), and the sequence was analyzed using QIIME2 (version 2020.2). The quality of the joined sequence was filtered using the q2-demux plugin, noise was removed using DADA2, OTUs were clustered, and a function table for analysis was generated. The processed sequence data was assigned to and aligned with the Green genes reference database at 99% sequence similarity. LEfSe was applied to determine a classification group having the highest discrimination degree in the group based on the feature table. For statistical analysis and visual inspection, the OTU table including the classification group in the plain format and the metadata file was analyzed using microbiomeAnalyst. The functional possibility of the microbiota data of different groups was predicted based on the 16S rRNA data using Piphillin, which was established by assigning the functional characteristics using the KEGG database and the BioCyc reference database.

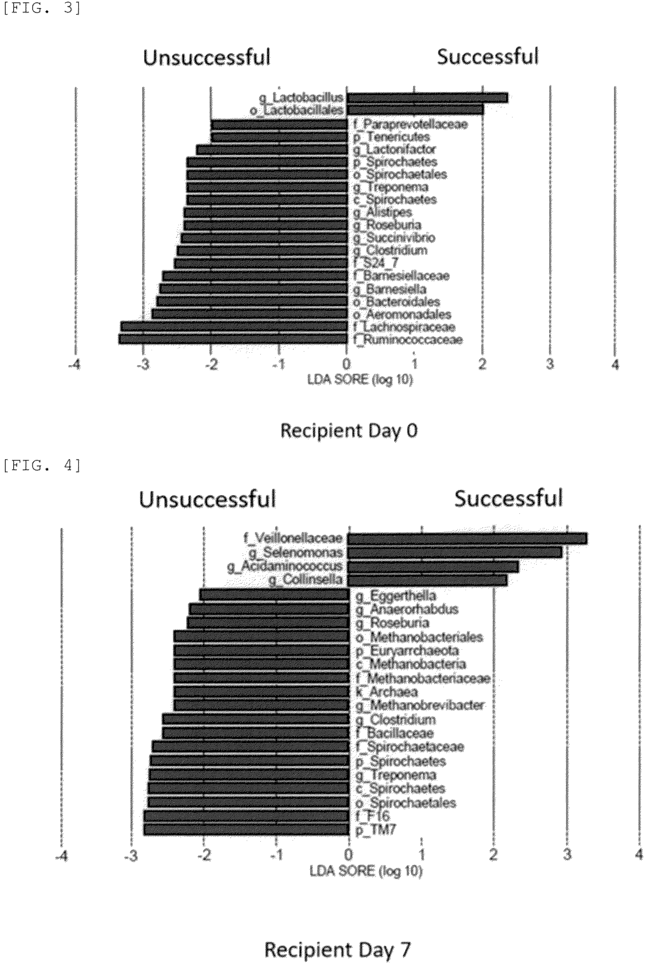

FIGS. 2 to 4 show the analysis results of the types of bacteria. FIG. 2 shows the analysis results of the donor before FMT implementation (day 0), FIG. 3 shows the analysis results of the recipient before FMT implementation (day 0), and FIG. 4 shows the analysis results of the recipient after 7 days of FMT. In the success group (Successful) in FIGS. 2 to 4, it can be said that the bacteria are characterized by the FMT success as the bacteria has a high LDA score. In addition, even in the failure group, it can be said that the bacteria are characterized by the FMT failure as the bacteria has a high LDA score. It should be noted that the bacteria shown in FIGS. 2 to 4 are exemplified by particularly extracting bacteria having a high LDA score, and all the bacteria are not described in both the success group and the failure group.

As shown in FIG. 2, in a case where the donors of the success group and the failure group of FMT are compared, the class Bacteroidia (No. 1), the family Prevotellaceae (No. 2), the genus Prevotella (No. 3), the genus Succinispira (No. 4), the genus Selenomonas (No. 5), the genus Gemmiger (No. 6), and the genus Odoribacter (No. 7) had a high LDA score. On the other hand, in the failure group, the LDA scores of the genus Lactonifactor (No. 23), the genus Alistipes (No. 22), the genus Roseburia (No. 21), and the like were high. Details of the bacteria included in the failure group are described in Bacterial Classification Nos. 14 to 23.

As shown in FIG. 3, before FMT implementation, in the recipients of the success group, the genus Lactobacillus (No. 8) and the order Lactobacillales (No. 9) had a high LDA score. On the other hand, in the recipients of the failure group, before FMT implementation, the family Ruminococcaceae (No. 40), the family Lachnospiraceae (No. 39), and the like had a high LDA score. Details of the bacteria included in the failure group are described in Bacterial Classification Nos. 24 to 40.

As shown in FIG. 4, after 7 days of FMT implementation, in the recipient of the success group, the family Veillonellaceae (No. 10), the genus Selenomonas (No. 5), the genus Acidaminococcus (No. 11), and the genus Collinsella (No. 12) had a high LDA score. On the other hand, in the recipient of the failure group, after 7 days of FMT implementation, the phylum TM7 (No. 58), the family F16 (No. 57), and the like had a high LDA score. Details of the bacteria included in the failure group are described in Bacterial Classification Nos. 41 to 58.

From the results of FIGS. 2 to 4, it was possible to specify 13 types of bacteria that are highly likely to lead to the success of FMT from the donors of the success group and the recipients of the success group. Among these, since the genus Selenomonas (No. 5) showed a high LDA score in both the donors of the success group and the recipients of the success group (Day 7), it was possible to specify the genus Selenomonas (No. 5) as a bacterium that is highly likely to lead to the success of FMT. In a case where a therapeutic agent for diarrhea is prepared, in a case where feces is used as a base, as the cultured genus Selenomonas (No. 5) is added or the cultured genus Selenomonas (No. 5) is used, the genus Selenomonas (No. 5) is useful for preparing a therapeutic agent for diarrhea. On the other hand, the phylum Tenericutes (No. 16) and the phylum Spirochaetes (No. 20) were discovered in both the donors of the failure group and the recipients (Day 0) of the failure group. Therefore, in a case where a therapeutic agent for diarrhea is prepared, it is preferable that the therapeutic agent for diarrhea does not contain or contains the phylum Tenericutes (No. 16) and the phylum Spirochaetes (No. 20) in a very small amount. In addition, from the results shown in FIG. 4, it can be said that the therapeutic agent for diarrhea disclosed in the present application can be a therapeutic agent that increases at least one type of bacterium selected from the group consisting of the family Veillonellaceae (No. 10), the genus Selenomonas (No. 5), the genus Acidaminococcus (No. 11), and the genus Collinsella (No. 12) contained in the feces of a recipient after administration.

### <Example 3>

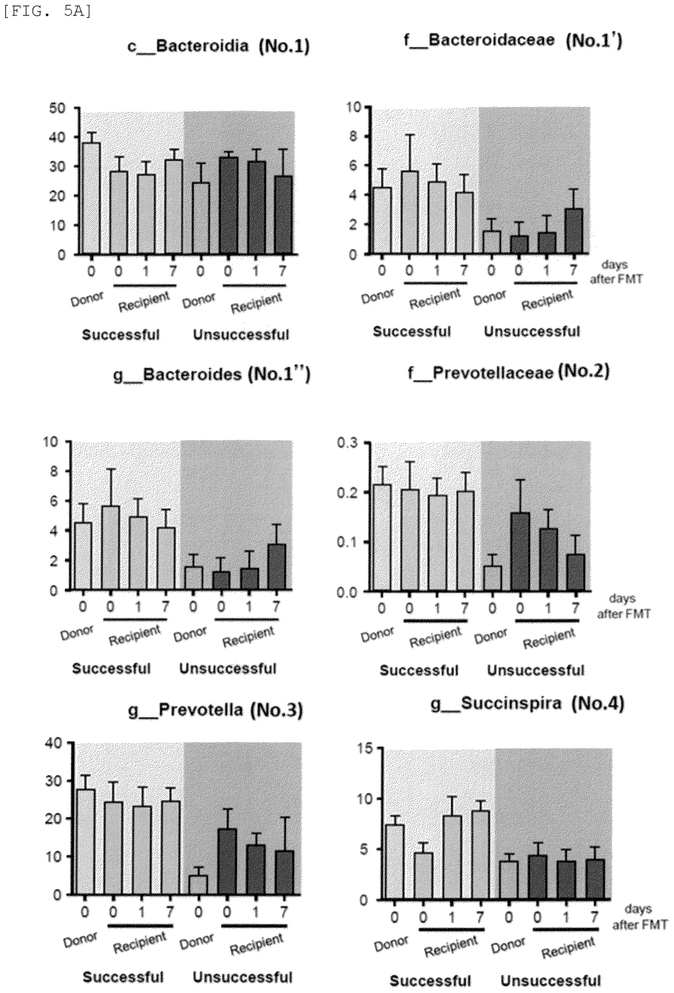

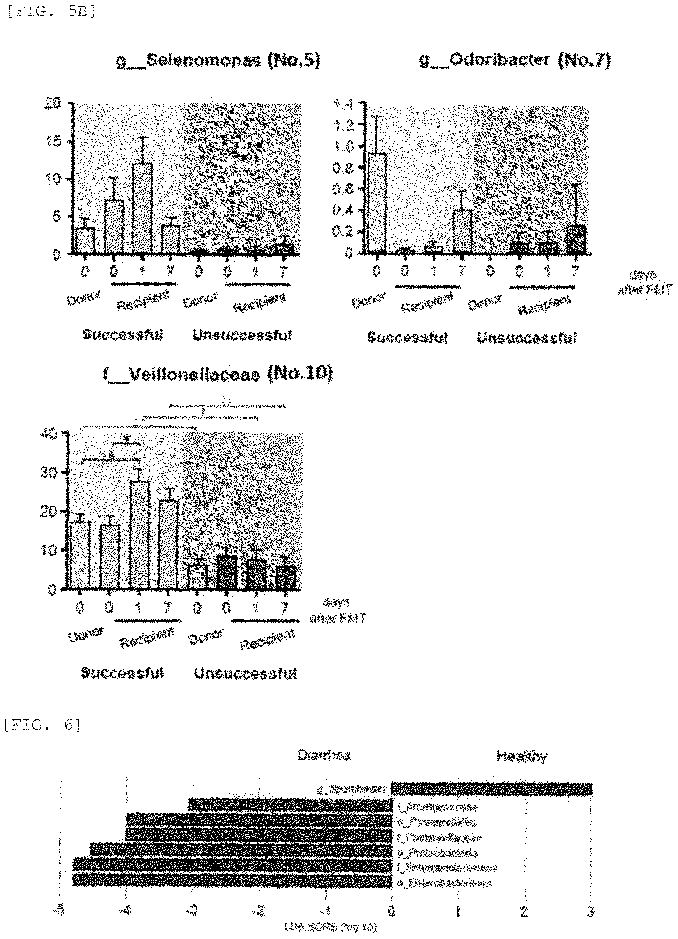

Subsequently, for some of the 13 types of bacteria that lead to the success of FMT, which were obtained by statistical analysis in Example 2, it was further confirmed whether the bacteria were actually useful for FMT in 20 cases. FIG. 5 shows relative abundance (relative abundance: % as a numerical value on the vertical axis) of each bacterium in all bacteria in the feces collected from the donor and the recipient (day 0, after 1 day, and after 7 days) of the success group, and relative abundance of each bacterium in all bacteria in the feces collected from the donor and the recipient (day 0, after 1 day, and after 7 days) of the failure group. The relative abundance shown in FIG. 5 is a ratio of the number of copies of each bacterium to the number of copies of 16S rRNA of all bacteria, and corresponds to a ratio (occupancy rate) of the number of each bacterium to the number of all bacteria in the feces.

As is apparent in FIG. 5, all the bacteria listed in Nos. 1 to 5, 7, and 10 were contained in the success group more than the failure group (in particular, 7 days after the donor and the recipient). Among these, the occupancy rate of the genus Selenomonas in both donors and recipients of the success group was much higher than that in both donors and recipients of the failure group. In addition, regarding the donor, the occupancy rate of the bacteria included in the success group was higher than that of the failure group in any of the bacteria. Therefore, in a case where a therapeutic agent for diarrhea is prepared, it is preferable to increase the occupancy rate of the bacteria of Nos. 1 to 5, 7, and 10. Although not limited, examples thereof are considered to include (1) increasing the occupancy rate (average value) of the bacteria included in the donor of the failure group, (2) an average value of the occupancy rates of the failure group and the success group or more, and (3) an average value of the occupancy rates of the success group or more. The results shown in FIG. 5 are numerical values calculated based on the above (1) to (3), which are described below. Although the numerical values of the following (1) are described as "or more", since the significant figures are rounded up to the second decimal point, the numerical values described in the following (1) are described as "or more", but the numerical values are larger than the occupancy rate (average value) of the bacteria included in the donor of the failure group.

· No. 1 (c_Bacteroidia): (1) 24.21% or more, (2) 31.34% or more, (3) 38.47% or more.
· No. 2 (f_Prevotellaceae) : (1) 4.43% or more, (2) 12.64% or more, (3) 20.85% or more.
· No. 3 (g_Prevotella): (1) 4.77% or more, (2) 15.89% or more, and (3) 27.00% or more.
· No. 4 (g_Succinispira) : (1) 4.12% or more, (2) 5.75% or more, (3) 7.40% or more.
· No. 5 (g_Selenomonas): (1) 0.09% or more, (2) 1.78% or more, and (3) 3.47% or more.
· No. 7 (g_Odoribacter): (1) 0% or more (greater than 0), (2) 0.46% or more, (3) 0.92% or more.
· No. 10 (f_Veillonellaceae): (1) 4.86% or more, (2) 10.32% or more, (3) 15.78% or more.

Since No. 1 was at the class level, the analysis at the family level (No. 65, f_Bacteroidaceae) and the genus level (No. 66, g_Bacteroides) was performed separately. Table 6 shows DNA sequences encoding 16S rRNA used for the analysis. In addition, FIG. 5 shows an analysis result (No. 1') of f_Bacteroidaceae and an analysis result (No. 1'') of g_Bacteroides.

As is apparent from No. 1' and No. 1'' of FIG. 5, even at the family level and the genus level, which are lower classifications of No. 1 (c_Bacteroidia), occupancy rates of the donors and the recipients included in the success group was higher than those of the donors and the recipients included in the failure group. Numerical values of the occupancy rates calculated based on the above (1) to (3) are described below.
· No. 1' (f_Bacteroidaceae): (1) 1.48% or more, (2) 2.94% or more, (3) 4.40% or more.
· No. 1'' (g_Bacteroides): (1) 1.48% or more, (2) 2.94% or more, and (3) 4.40% or more.

**[Table 6]**

| Bacterial Classification No. | Bacterial name | SEQ ID NO | DNA sequence encoding 16S rRNA |
|---|---|---|---|
| 65 | f_Bacteroidaceae | 65 | AGCGCTGTTTGATACGCGCACTTTCGAGCGTCAACGTGAGTGGCGGCTTAGCAGGCTGCCTTCGCAATCGGGGTTCTTCGTCAGATCTAAGCATTTCACGCGCTAGACCACGAATTGCGCCTGCCTCAAGCGTA CTGCAGGTGCGAGTTGCAGTTGCAGCTTGAGGTTGAGGCGCAAACTTTCAGAGCTGACTTAAAACGCGTCTACGCTGCCTTTAAGCCAACAAAGCGGAGAGCGTCGGCATCCTCGGTATGCGCGGGTG CTGGGACGGAGTTAGCGGAGGCTTATTCGTTTACGGTACATGCAAAACCTACACGTAGGTTCGTTTTGTTCGCGCAAAAGAATTTACAACCGCGTAGGGCGTGATCGTTTCACGCTAGTTGGCTGGTTCAGGC TCTCGGCCATTGACCAATATTGCTCA |
| 66 | g_Bacteroides | 66 | |

### <Example 4>

Examples 2 and 3 described above are statistical analysis results based on the donors and recipients who were subjected to FMT. In order to further search for bacteria that lead to the success of FMT, in Example 4, the analysis was performed including fecal samples of healthy cows (n = 109) and cows with diarrhea (n = 49) that were not subjected to FMT in addition to the donor (success group + failure group) and the recipient (success group + failure group) that were subjected to FMT. The specific procedure is the same as in Examples 2 and 3.

FIG. 6 shows results of the LefSe analysis. As shown in FIG. 6, in the comparison between the healthy cow and the cow with diarrhea, the genus Sporobacter (No. 13) had a high LDA score. On the other hand, in the cow with diarrhea, the family Enterobacteriaceae (No. 64), the order Enterobacteriales (No. 63), and the like had a high LDA score. Details of the bacteria contained in the cow with diarrhea are described in Bacterial Classification Nos. 59 to 64.

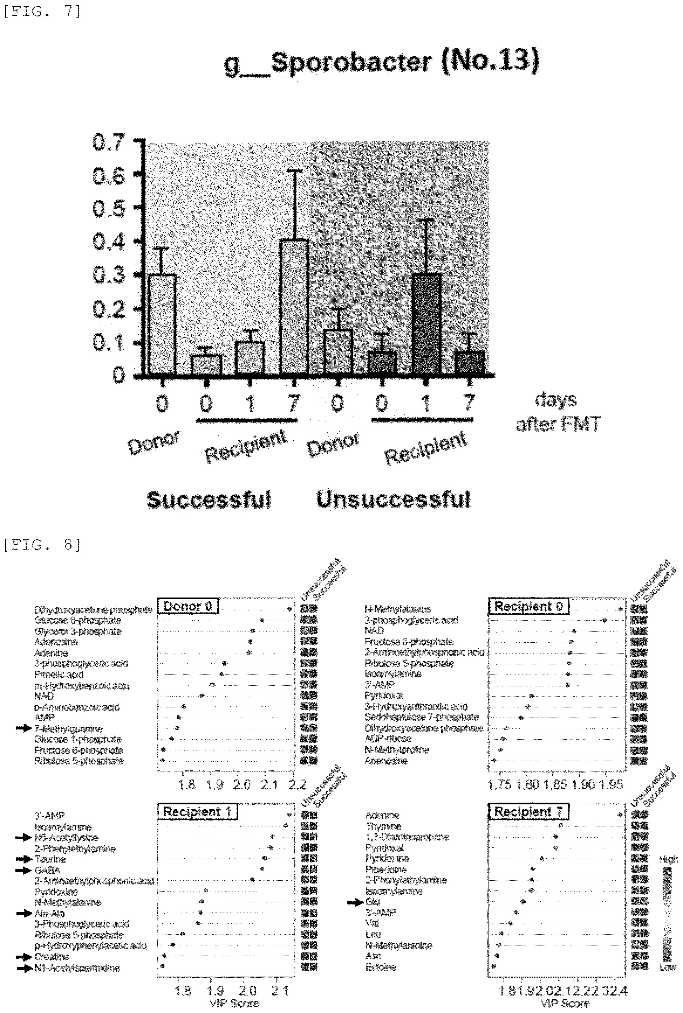

Subsequently, the ratio of the genus Sporobacter (No. 13) actually contained in the donor and the recipient that were subjected to FMT was examined. FIG. 7 shows the results. Meanwhile, it is desirable that the bacterium that leads to the success of FMT is a bacterium that is (1) contained in a large amount in healthy cows and in a small amount in cows with diarrhea, (2) contained in a large amount in donors of the success group and in a small amount in donors of the failure group, and (3) contained in a very small amount in recipients (day 0) of the success group (that is, occupancy rate recipient before administration of a therapeutic agent for diarrhea is small) and also contained in a small amount in recipients (day 0) of the failure group. As is apparent from FIG. 7, the genus Sporobacter (No. 13) does not necessarily have a large occupancy rate in feces compared with the bacteria searched in Example 3, but since the genus Sporobacter (No. 13) has all of preferable conditions that lead to the success of FMT, it is desirable that the genus Sporobacter (No. 13) is included in the therapeutic agent for diarrhea as a super donor. In a case where the genus Sporobacter (No. 13) is included, the genus Sporobacter (No. 13) is preferably included in the therapeutic agent for diarrhea to be the following ratio or more, when the calculation is performed in the same manner as in Example 3.
· No. 13 (g_Sporobacter) : (1) 0.16% or more, (2) 0.21% or more, (3) 0.28% or more.

As a result described above, it was confirmed that the bacteria listed in Bacterial Classification Nos. 1 to 13 can lead to the success of FMT, and among these, the genus Selenomonas (No. 5) and the genus Sporobacter (No. 13) are preferable. Therefore, it is expected that the therapeutic effect of FMT will be further enhanced by incorporating a large amount of the genus Selenomonas (No. 5) and/or the genus Sporobacter (No. 13) in the therapeutic agent for diarrhea. It is known that the genus Selenomonas is involved in ATP production through the influence on a succinic acid-propionic acid pathway. It is assumed that the improvement of energy metabolism due to the presence of the genus Selenomonas is involved in the therapeutic effect on diarrhea.

### <Example 5>

### [CE-TOFMS metabolomics analysis]

Subsequently, metabolites contained in the fecal sample were analyzed. The metabolomics analysis using a fecal sample was carried out by Human Metabolome Technologies, Inc. Specifically, the sample was dried using a freeze dryer (TAITEC) and analyzed with CE-TOFMS (Agilent). The identified metabolites were quantified by comparing the measured peak area with a standard (known metabolite) peak area using ChemStation Software (Agilent Technologies).

FIG. 8 shows the VIP score of the metabolite analyzed by CE-TOFMS metabolomics analysis. The metabolites indicated by the arrow in FIG. 8 are metabolites specific to the success group, and the metabolites not indicated by the arrow are metabolites specific to the failure group. As shown in "Donor 0" in FIG. 8, in the donor of the success group, the VIP score of 7-methylguanine was high. As shown in "Recipient 1" of FIG. 8, after 1 day of FMT implementation, in the recipient of the success group, the VIP scores of N6-acetyllysine, Taurine, γ-aminobutyric acid (GABA), Ala-Ala, Creatine, and N1-acetylspermidine were high. In addition, as shown in "Recipient 7" of FIG. 8, after 7 days of FMT implementation, in the recipient of the success group, the VIP score of glutamic acid (Glu) was high.

It is not clear whether the metabolite is directly involved in the treatment of diarrhea. However, since it is a fact that the metabolites shown in FIG. 8 are contained in a large amount in the success group, it is expected that the therapeutic effect on diarrhea will be further enhanced by adding at least one selected from the metabolites to the therapeutic agent for diarrhea.

### <Example 6>

[Difference in effect of FMT by freeze-dried and frozen fecal microorganisms (therapeutic agent for diarrhea)]

Subsequently, in order to confirm the difference in the effect in a case where FMT was performed using the freeze-dried fecal microorganisms and the frozen fecal microorganisms, the experiment was performed according to the following procedure.

### 1. Adjustment of fecal microorganisms

(1) Feces (25 g) collected from a healthy cow (donor) was suspended in 125 ml of cold-PBS.
(2) The mixture was stirred with a hand mixer for 2 minutes.
(3) The mixture was filtered using a 0.5 mm pore nylon mesh.
(4) The adjustment was carried out at a ratio of 3:1 (filtered product of (3) : Nycodenz) using a multi-purpose density gradient centrifugal separation medium (Nycodenz).
(5) The mixture was rapidly centrifuged at 10,000 g and 4°C for 40 minutes.
(6) The microbial fraction was recovered (a part thereof was collected for metagenomic analysis).
(7) Fecal microorganisms were prepared by performing the following treatments on the microbial fraction recovered in the above (6).
   - Storing at -80°C (frozen fecal microorganism)
   - Drying for 6 hours using a freeze-drying device (manufactured by TAITEC Corporation, model: VD-550R) (freeze-dried fecal microorganisms)

### 2. Implementation of FMT

The frozen fecal microorganisms and the freeze-dried fecal microorganisms adjusted in the above 1. were suspended in 100 ml of PBS and transplanted into a cow with diarrhea (recipient) via the anus.

### 3. Analysis

Feces were collected from the recipients immediately before transplantation (D0), the day after transplantation (D1), and one week after transplantation (D7), and metagenomic analysis was performed in the same manner as in Example 2 and metabolomics analysis was performed in the same manner as in Example 5.

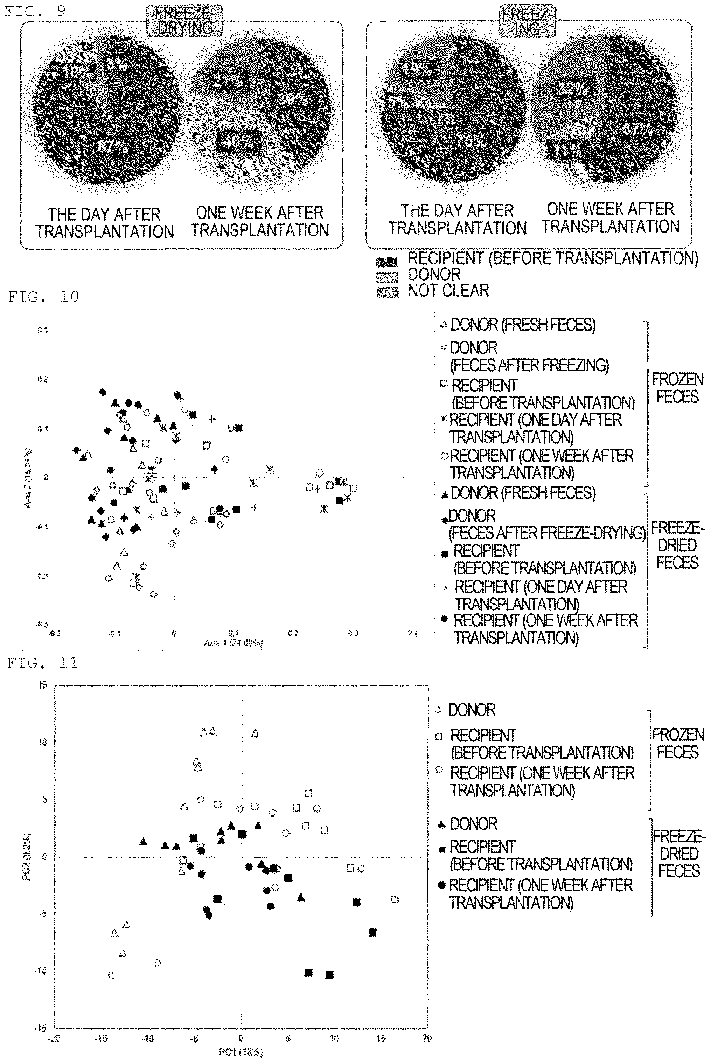

FIG. 9 shows a summary of the results of the metagenomic analysis. The white arrow in FIG. 9 shows a match rate between the intestinal bacterial flora of the recipient and the donor one week after transplantation. As is apparent from FIG. 9, in a case where the freeze-dried fecal microorganisms were used, the proportion of the intestinal bacterial flora of the recipient changed to the intestinal bacterial flora of the donor was about 4 times as large as that in a case where the frozen fecal microorganisms were used. FMT is intended to bring the intestinal bacterial flora of the recipient close to the intestinal bacterial flora of the donor. Therefore, it can be said that the freeze-dried fecal microorganisms exhibit a remarkable effect in a case where FMT is performed, as compared with the frozen fecal microorganisms.

FIG. 10 shows detailed results of the metagenomic analysis, and FIG. 11 shows detailed results of the metabolomics analysis. In addition, Table 7 shows p values of the metagenomic analysis shown in FIG. 10, and Table 8 shows p values of the metabolomics analysis shown in FIG. 11.

**[Table 7]**

| **Transplantation test** | **Comparison (fresh feces)** | **p value** |
|---|---|---|
| Frozen feces | D vs R0 | 0.007 |
| | D vs R1 | 0.003 |
| | D vs R7 | 0.048 |
| Freeze-dried feces | D vs R0 | 0.001 |
| | D vs R1 | 0.001 |
| | D vs R7 | **0.096** |

| **Transplantation test** | **Comparison (feces after freezing)** | **p value** |
|---|---|---|
| Frozen feces | D vs R0 | 0.009 |
| | D vs R1 | 0.006 |
| | D vs R7 | 0.006 |
| Freeze-dried feces | D vs R0 | 0.003 |
| | D vs R1 | 0.008 |
| | D vs R7 | **0.096** |

**[Table 8]**

| **Transplantation test** | **Comparison (fresh feces)** | **p value** |
|---|---|---|
| Frozen feces | D vs R0 | 0.0007 |
| | D vs R7 | 0.0178 |
| Freeze-dried feces | D vs R0 | 0.0253 |
| | D vs R7 | **0.6719** |

As shown in Tables 7 and 8, only the feces collected from the recipient one week after the transplantation using the freeze-dried fecal microorganisms was not significantly different from the donor (fresh feces, frozen feces) (p value of 0.096 in Table 7 and p value of 0.6719 in Table 8). In other words, this means that only the intestinal bacterial flora and the metabolite of the recipient one week after the transplantation using the freeze-dried fecal microorganisms have changed to be close to the intestinal environment of the donor to an extent that the intestinal bacterial flora and the metabolite of the recipient cannot be distinguished from the intestinal bacterial flora and the metabolite of the donor.

As a result, it was confirmed that in a case where FMT is performed using the fecal microorganisms collected from a donor, the effect on the intestinal bacterial flora and the metabolite of the recipient is remarkably excellent in a case where freeze-dried fecal microorganisms are used as compared with the frozen fecal microorganisms.

In addition, since the freeze-dried fecal microorganisms can be stored at room temperature, the handling thereof is extremely simple. However, since the frozen fecal microorganisms require equipment for storing at an extremely low temperature of -80°C and the like, there is a problem in that a place where FMT is performed is limited. In a case where the freeze-dried fecal microorganisms are used, as compared with the frozen fecal microorganisms, in addition to the above effect exhibited when FMT is performed, a different effect such as convenience of handling is simultaneously exhibited.

### Industrial Applicability

By using the therapeutic agent for diarrhea disclosed in the present application, bovine diarrhea can be treated. Therefore, the therapeutic agent for diarrhea disclosed in the present application is useful for the livestock industry.

## Claims

1. A therapeutic agent for diarrhea comprising:
bacteria,
wherein the therapeutic agent for diarrhea contains
at least one type of bacterium selected from the group consisting of 13 types of bacteria listed in Bacterial Classification Nos. 1 to 13 shown in Table 1 below, and
the 13 types of bacteria listed in Bacterial Classification Nos. 1 to 13 each contain a sequence having 95% or more sequence identity to a DNA sequence encoding 16S rRNA represented by SEQ ID NOs: 1 to 13.
**[Table 1]**
| SEQ ID NO | Bacteria | |
|---|---|---|
| | Bacterial Classification No. | Bacterial name |
| 1 | 1 | c_Bacteroidia |
| 2 | 2 | f Prevotellaceae |
| 3 | 3 | g_Prevotella |
| 4 | 4 | g_Succinispira |
| 5 | 5 | g_Selenomonas |
| 6 | 6 | g_Gemmiger |
| 7 | 7 | g_Odoribacter |
| 8 | 8 | g_Lactobacillus |
| 9 | 9 | ο_Lactobacillales |
| 10 | 10 | f_Veillonellaceae |
| 11 | 11 | g_Acidaminococcus |
| 12 | 12 | g_Collinsella |
| 13 | 13 | g_Sporobacter |

2. The therapeutic agent for diarrhea according to Claim 1,
wherein the bacteria include at least one type selected from the group consisting of Bacterial Classification Nos. 1 to 5, 7, 10, and 13, and has an occupancy rate shown in Table 2 below.
**[Table 2]**
| SEQ ID NO | Bacteria | | Occupancy rate |
|---|---|---|---|
| | Bacterial Classification No. | Bacterial name | |
| 1 | 1 | c_Bacteroidia | 24.21% or more |
| 2 | 2 | f Prevotellaceae | 4.43% or more |
| 3 | 3 | g_Prevotella | 4.77% or more |
| 4 | 4 | g_Succinispira | 4.12% or more |
| 5 | 5 | g_Seienomonas | 0.09% or more |
| 7 | 7 | g_Odoribacter | Greater than 0% |
| 10 | 10 | f Veillonellaceae | 4.86% or more |
| 13 | 13 | g_Sporobacter | 0.16% or more |

3. The therapeutic agent for diarrhea according to Claim 1, comprising:
at least g_Sporobacter of Bacterial Classification No. 13.

4. The therapeutic agent for diarrhea according to Claim 2, comprising:
at least g_Sporobacter of Bacterial Classification No. 13.

5. The therapeutic agent for diarrhea according to Claim 4,
wherein an occupancy rate of g_Sporobacter of Bacterial Classification No. 13 is 0.21% or more.

6. The therapeutic agent for diarrhea according to Claim 1, comprising:
at least g_Selenomonas of Bacterial Classification No. 5.

7. The therapeutic agent for diarrhea according to Claim 2, comprising:
at least g_Selenomonas of Bacterial Classification No. 5.

8. The therapeutic agent for diarrhea according to Claim 7,
wherein an occupancy rate of g_Selenomonas of Bacterial Classification No. 5 is 1.78% or more.

9. The therapeutic agent for diarrhea according to any one of Claims 1 to 8, which is used for treatment of bovine diarrhea.

10. The therapeutic agent for diarrhea according to any one of Claims 1 to 8, further comprising:
at least one type of compound selected from the group consisting of 7-methylguanine, N6-acetyllysine, Taurine, GABA, Ala-Ala, Creatine, N1-acetylspermidine, and Glu.

11. The therapeutic agent for diarrhea according to any one of Claims 1 to 8,
wherein the therapeutic agent for diarrhea is to increase at least one type of bacterium selected from the group consisting of g_Selenomonas of Bacterial Classification No. 5, f_Veillonellaceae of Bacterial Classification No. 10, g_Acidaminococcus of Bacterial Classification No. 11, and g_Collinsella of Bacterial Classification No. 12, which are contained in feces of a recipient after administration.

12. The therapeutic agent for diarrhea according to any one of Claims 1 to 8,
wherein the therapeutic agent for diarrhea is in a freeze-dried form.

13. The therapeutic agent for diarrhea according to Claim 9,
wherein the therapeutic agent for diarrhea is in the freeze-dried form.

14. A method for treating bovine diarrhea, the method comprising:
a step of administering the therapeutic agent for diarrhea according to any one of Claims 1 to 8 into a bovine intestinal tract.

15. A method for treating bovine diarrhea, the method comprising:
a step of administering the therapeutic agent for diarrhea according to Claim 12 into a bovine intestinal tract.
